(19) Europäisches Patentamt
European Patent Office
Office européen des brevets

(11) **EP 4 462 435 A1**

(12) **EUROPEAN PATENT APPLICATION**

(43) Date of publication:
**13.11.2024 Bulletin 2024/46**

(21) Application number: **24160954.4**

(22) Date of filing: **01.03.2024**

(51) International Patent Classification (IPC):
**G16B 15/00** (2019.01)   **G16B 40/20** (2019.01)
**G16C 20/30** (2019.01)   **G16C 20/70** (2019.01)

(52) Cooperative Patent Classification (CPC):
**G16B 15/00; G16B 40/20; G16C 20/30;
G16C 20/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.05.2023   JP 2023078036**

(71) Applicant: **Hitachi, Ltd.**
**Tokyo 100-8280 (JP)**

(72) Inventors:
• **SHOJI, Tatsuma**
  **Tokyo, 100-8280 (JP)**
• **TAKEUCHI, Wataru**
  **Tokyo, 100-8280 (JP)**

(74) Representative: **MERH-IP Matias Erny Reichl
Hoffmann
Patentanwälte PartG mbB
Paul-Heyse-Strasse 29
80336 München (DE)**

(54) **SYSTEMS AND METHODS FOR SIMULATION OF BIOPOLYMER STABILITY**

(57)     Provided is a system capable of predicting, with high accuracy, the degradation stability of biopolymers having an arbitrary sequence. A computer acquires information of a sequence of the biopolymer, classifies the sequence into a plurality of regions, calculates stability of each of the plurality of regions, and executes predictive simulation of the stability of the biopolymer based on a representative value as a representation of the stability of each of the plurality of regions based on a result of the calculation.

FIG. 1

**EP 4 462 435 A1**

**Description**

CROSS-REFERENCE TO PRIOR APPLICATION

This application relates to and claims the benefit of priority from Japanese Patent Application number 2023-078036, filed on May 10, 2023 the entire disclosure of which is incorporated herein by reference.

BACKGROUND

The present invention relates to a system and method for simulating the stability of biopolymers using a computer.
Biopolymers exist in living organisms and are polymers composed of polymerized monomer units. As biopolymers, known are polynucleotides, polypeptides, and polysaccharides. Because biopolymers are known to have diverse correlations with polymeric structures and pharmacological activities, when developing biopolymers as pharmaceutical products, computers are often used to evaluate the structure and drug efficacy of biopolymers. For example, it is known that computers can be used to predict the stability of biopolymers against degradation in the body.
NPTL 1 describes predicting the half-life of mRNA of an arbitrary sequence by defining, as half-life, the stability of mRNA as a biopolymer, measuring the half-life of mRNA of numerous genes based on RNA-Seq, and applying machine learning thereto.
[NPTL 1]
Medina-Munoz, Santiago Gerardo, et al. "Crosstalk between codon optimality and cis-regulatory elements dictates mRNA stability" Genome biology 22.1 (2021):1-23

SUMMARY

Nevertheless, with the conventional method described as the patent literature described above, in effect, there is a problem in that the correlation coefficient between the predicted value of mRNA stability and its measured value is small. Thus, an object of the present invention is to provide a system capable of predicting, with high accuracy, the degradation stability of biopolymers having an arbitrary sequence.
In order to achieve the foregoing object, the present invention is a computer system which simulates stability of a biopolymer, comprising: a controller that executes a program stored in a memory, wherein the controller: acquires information of a sequence of the biopolymer; classifies the sequence into a plurality of regions; calculates stability of each of the plurality of regions; and executes predictive simulation of the stability of the biopolymer based on a representative value as a representation of the stability of each of the plurality of regions based on a result of the calculation. The present invention additionally relates to a method of simulating stability of a biopolymer.
According to the present invention, it is possible to provide a system capable of predicting, with high accuracy, the degradation stability of biopolymers having an arbitrary sequence.

BRIEF DESCRIPTION OF DRAWINGS

[Fig. 1]

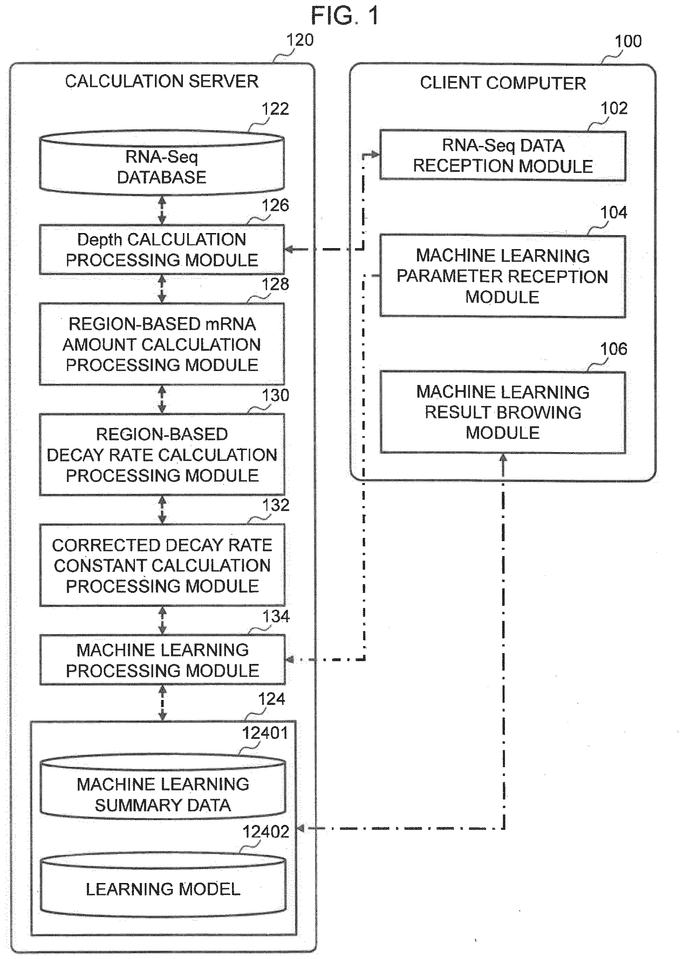

Fig. 1 is a block diagram of an embodiment of a computer system according to the present invention that simulates the stability of biopolymers.
[Fig. 2]

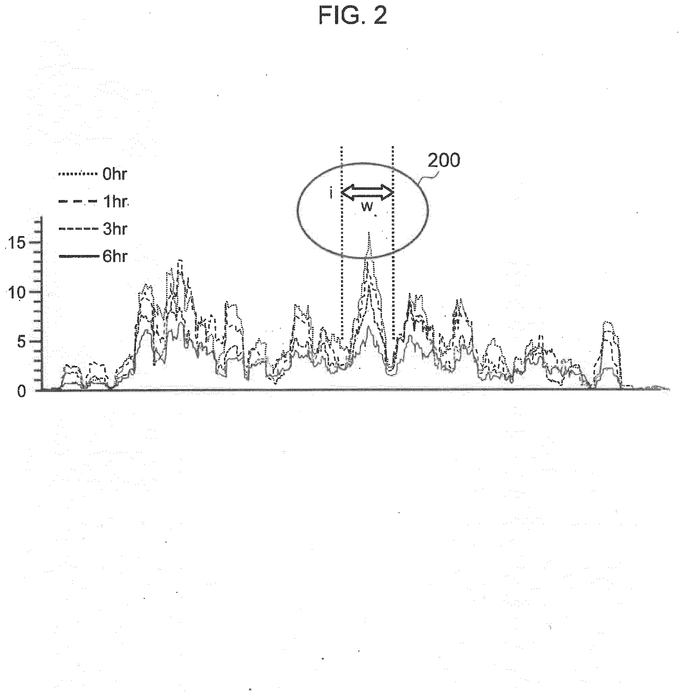

Fig. 2 is a graph schematically showing the differences in the stability for each region of mRNA configured from a prescribed base sequence.
[Fig. 3]

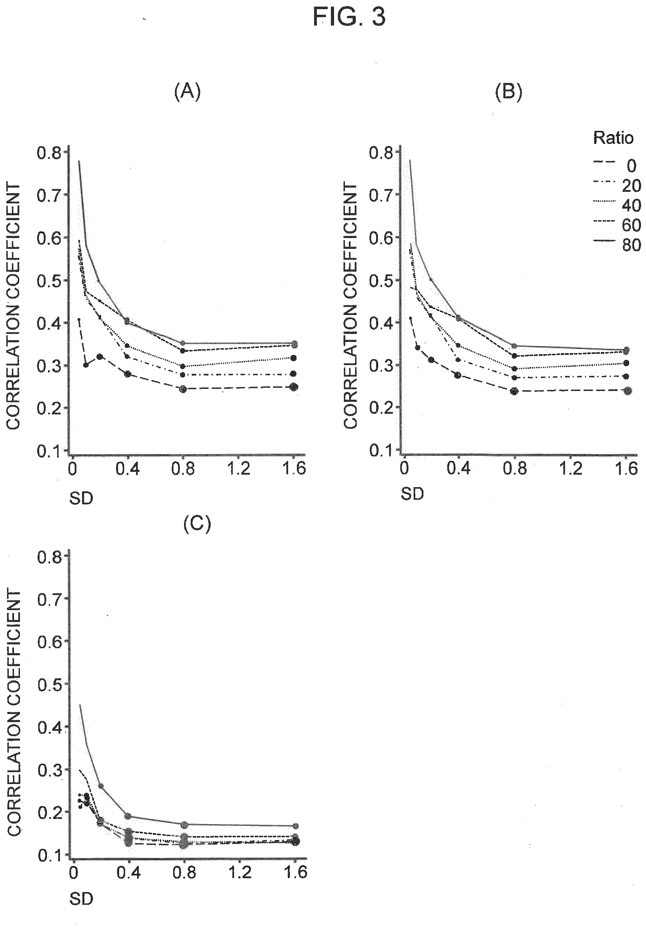

Fig. 3 is a graph that compares the correlation coefficient in the stability prediction of mRNA between the present invention and the conventional method.
[Fig. 4]

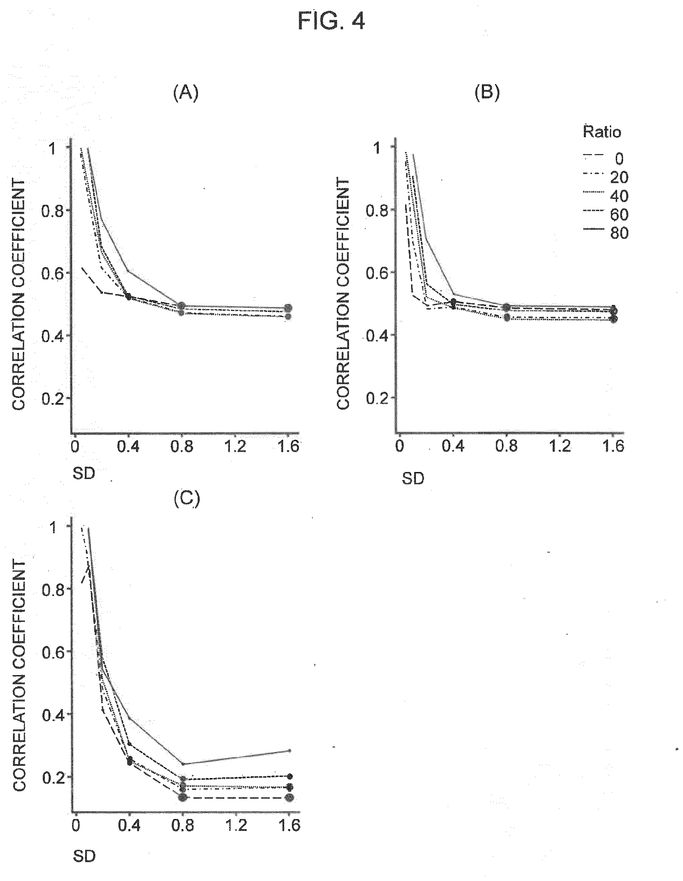

Fig. 4 is another graph that compares the correlation coefficient in the stability prediction of mRNA between the present invention and the conventional method.
[Fig. 5]

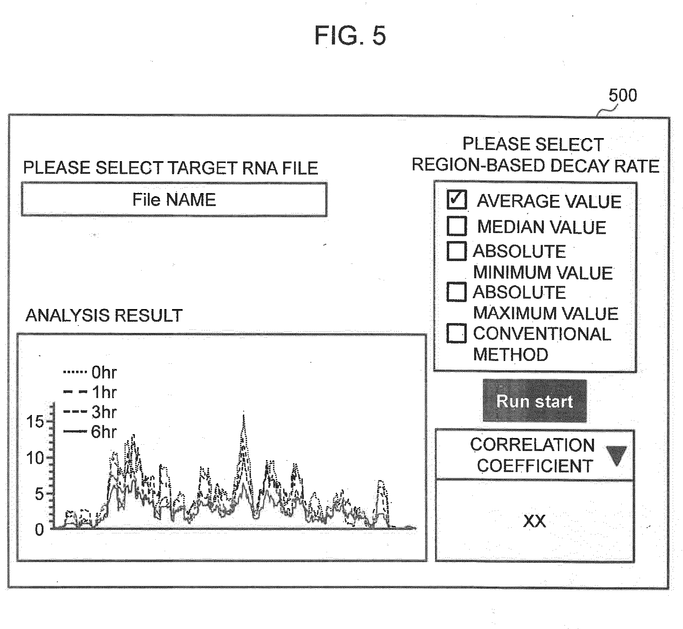

Fig. 5 is a screen example that is displayed via a computer system according to the present invention that simulates the stability of biopolymers.
[Fig. 6]

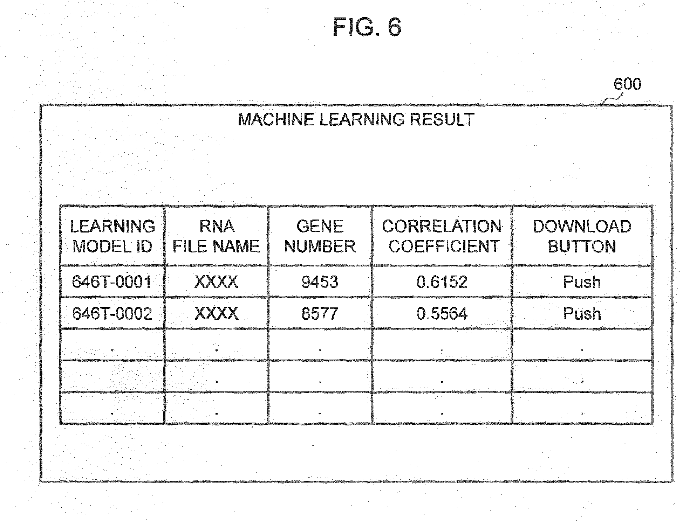

Fig. 6 is another screen example that is displayed via a computer system according to the present invention that simulates the stability of biopolymers.

## DESCRIPTION OF EMBODIMENTS

An embodiment of the present invention is now explained. Fig. 1 is a block diagram of an embodiment of a computer system according to the present invention that simulates the stability of biopolymers. The computer system comprises a calculation server 120, and a client computer 100 connected to the calculation server. The computer system's controller (or processor) executes, based a program stored in the memory, the calculation of predicting the stability of a biopolymer based on a sequence of a plurality of monomers configuring the biopolymer in order to simulate the stability of the biopolymer. Before explaining the system of Fig. 1, the new knowledge of the present inventors in a simulation system of predicting the stability of biopolymers is foremost explained.

As described above, biopolymers exist in living organisms and are polymers composed of polymerized monomer units, and known as biopolymers are polynucleotides, polypeptides, and polysaccharides. In the following explanation, as the biopolymer, mRNA is taken as an example among RNAs as polynucleotides.

There is a plurality of nucleotide sequences, or base sequences, that code one amino acid. Accordingly, there are an extremely large number of mRNA sequences that code the same protein. In the development of oligonucleotide therapeutics such as mRNA vaccines, it is necessary to select mRNA molecules that are stable against degradation in the living organism from numerous sequence groups of mRNA that code the target protein.

The half-life of mRNA explained in the conventional method of NPTL 1 described above is, in effect, used to mean a decay rate constant (decay rate of the conventional method is hereinafter referred to as "$\beta_g^{(S)}$"), and this may otherwise be referred to as a "degradation rate constant" or a "stability constant". The decay rate constant $\beta_g^{(S)}$ of mRNA of the gene g is an index of mRNA stability as with half-life, and the decay rate constant $\beta_g^{(S)}$ is indicated in the mRNA decay (degradation) reaction model of Math (1) below.

In Math (1), t is time, and [mRNA$_g$] is the time series data of the intracellular mRNA amount of the gene g at time t. From Math (2) obtained by integrating Math (1), $\beta_g^{(S)}$ can be estimated by regressing log[mRNA$_g$] overtime. $\alpha_g^{(S)}$ of Math (2) is an integration constant or an intercept.

[Math 1]

$$\frac{d[\mathrm{mRNA}_g]}{dt} = \beta_g^{(S)}[\mathrm{mRNA}_g] \qquad (1)$$

[Math 2]

$$\log[\mathrm{mRNA}_g] = \beta_g^{(S)}t + \alpha_g^{(S)} \qquad (2)$$

Transcripts Per Million (TPM) is used to obtain [mRNA$_g$]. TPM is one index of the mRNA amount obtained from a fastq file, which is experimental data of RNA-Seq, and is a value in which the number of reads mapped to the gene is corrected based on the length of the reads, length of the gene, and total number of reads.

Specifically, after checking the quality of the fastq file using Trimmomatic (v0.39), performing mapping to the genome using HISAT2 (v2.2.1), and aggregating the number of reads for each gene based on the feature Count of Subread (v2.0.0), TPM is calculated based on a custom Python (3.9.15) script.

As the reference files used in the mapping and the Gene Transfer Format (GTF) file required for the aggregation of the number of reads, the latest versions (Release 109) as of January 2023 in Ensemble (https://asia.ensembl.org/index.html) were adopted.

For the experimental data of RNA-Seq, (human: GSE126520, mouse: GSE99978, zebrafish: SRP072296, African clawed frog: GSE65785) were selected, downloaded from Gene Expression Omnibus (GEO) (https://www.ncbi.nlm.nih.gov/geol) of the National Center for Biotechnology Information (NCBI), and used. Based on the foregoing operation, [mRNA$_g$] and $\beta_g^{(S)}$ were obtained for a total of 97,804 genes.

mRNA molecules are long chain molecules, and, as a result of intense study by the present inventors, as shown in Fig. 2, it was discovered that mRNA stability differs for each region of the nucleotide base sequence (monomer sequence: horizontal axis). Fig. 2 is a graph schematically showing the differences in the stability (vertical axis) for each region of mRNA configured from a prescribed base sequence.

The horizontal axis of Fig. 2 shows the base sequence; that is, the i-th base sequence, and the vertical axis shows the intracellular mRNA amount of the gene g having the same nucleotide at the i-th position for each predetermined time period (0 to 6 hr). This shows that, as the value of the vertical axis is greater, the amount of mRNA molecules is greater, and as the value of the vertical axis is smaller, the amount of mRNA molecules is smaller. In other words, as the ratio of change relative to the time of value of the vertical axis is greater, the stability of mRNA molecules is higher, and as the ratio of change relative to the time of value of the vertical axis is smaller, the stability of mRNA molecules is lower. Based on Fig. 2, it can be understood that the stability of mRNA molecules is fluctuating considerably like an irregular sawtooth pattern for each region 200 defined by i and w (w is the width of the base sequence).

With the conventional method described above, since no consideration is given to the region-based stability of mRNA molecules, when a learning model of machine learning is used for stability prediction, there were limits to the prediction accuracy of the stability of mRNA molecules having an arbitrary base sequence. The computer system of the present invention classifies a biopolymer into a plurality of regions, and simulates the stability of the overall biopolymer by introducing the region-based stability (region-based decay rate) into the calculation process.

Region-based stability is the degree in which the nucleotide sequence of the same region is maintained by comparing the mRNA time series data (RNA-Seq data) before and after the relevant time. The stability of each of a plurality of regions of a biopolymer is calculated based on the rate at which the sequence of the corresponding region becomes decayed. As the rate at which the nucleotide sequence of the same region becomes decayed (decay rate or the like) is smaller, the stability of the region will be higher. As a result of obtaining a representative value by synthesizing, integrating or fusing the stability of one region across the entire region and using the representative value as the factor that influences the stability of mRNA itself, it is possible to estimate and predict the stability of the biopolymer itself even with a biopolymer having region dependency on stability.

Furthermore, the computer system of the present invention integrates the region-based stability (region-based decay rate) and calculates the representative value (corrected decay rate constant) as the stability of the overall biopolymer, and thereby constructs a learning model (prediction model) of machine learning by using the calculated representative value as an objective variable. The computer system can accurately predict the stability of the overall biopolymer by applying the sequence of the biopolymer to the learning model as an explanation variable.

The corrected decay rate constant (decay rate constant of the present invention in relation to the conventional method; this is hereinafter referred to as "$\beta_g^{(A)}$"), which is the corrected stability of mRNA of the gene g, is defined by a window size w as the width of two or more arbitrary base sequences with an arbitrary location i as the starting point, and is a representative value based on the statistics function of the decay rate calculated for each region extracted from the mRNA sequence; that is, by expanding the reaction order from the mRNA amount of 200 of Fig. 2 to other than 1. In the following explanation, the region-based RNA amount is referred to as [mRNA$_{g,i,w}$], and the calculated decay rate is referred to as the region-based decay rate: $b_{g,i,w}^{(A)}$.

When considering a primary reaction model by applying in particular the entire gene region as [mRNA$_{g,i,w}$], $b_{g,i,w}^{(A)}$ will coincide with the decay rate constant $\beta_g^{(S)}$ as with the conventional method. When the size of the gene g is $L_g$, the range of the starting point i (monomer position) will be 1 to Lg-1, the range of the window size w (region width) will be 2 to $L_g-i+1$, and the computer system calculates [mRNA$_{g,i,w}$] for each of $L_g(L_g-1)/2$ types of regions in total.

The computer system calculates $b_{g,i,w}^{(A)}$ (region-based decay rate) by regressing the obtained [mRNA$_{g,i,w}$] over time by giving consideration to an appropriate reaction order, and integrates the obtained $L_g(L_g-1)/$two $b_{g,i,w}^{(A)}$ into $\beta_g^{(A)}$ based on the statistics function f.

Since existing bioinformatics tools can be used to obtain the mRNA amount at an arbitrary point of mRNA (hereinafter referred to as the "Depth"), the system can obtain the mRNA amount of an arbitrary region ([mRNA$_{g,i,w}$]) by averaging or adding this in each region.

The region-based decay rate $b_{g,i,w}^{(A)}$ as the time change of [mRNA$_{g,i,w}$] is shown in the decay reaction model formula of Math (3), and t is the time, [mRNA$_{g,i,w}$] is the mRNA amount of the region 200 defined by i, w of the gene g at time t (region-based mRNA amount), and $p_{g,i,w}$ is the reaction order upon making an assumption of the decay reaction of the region defined by i, w of the gene g.

From Math (4) obtained by integrating Math (3), $b_{g,i,w}^{(A)}$ can be estimated by regressing the amount on the left-hand side of Math (4) over time. $\alpha_{g,i,w}^{(A)}$ of Math (4) is an integration constant or an intercept.

[Math 3]

$$\frac{d[\mathrm{mRNA}_g]}{dt} = b_{g,i,w}{}^{(A)}[\mathrm{mRNA}_{g,i,w}]^{p_{g,i,w}} \qquad (3)$$

[Math 4]

$$\begin{cases} \dfrac{[\mathrm{mRNA}_{g,i,w}]^{1-p_{g,i,w}}}{1-p_{g,i,w}} = b_{g,i,w}{}^{(A)}t + a_{g,i,w}{}^{(A)}\,(p_{g,i,w} \neq 1) \\ \log[\mathrm{mRNA}_{g,i,w}] = b_{g,i,w}{}^{(A)}t + a_{g,i,w}{}^{(A)}\,(p_{g,i,w} = 1) \end{cases} \qquad (4)$$

Meanwhile, with this method, the residual error in the regression calculation at the time of calculating the corrected decay rate constant $\beta_g{}^{(A)}$ (= difference between the measured value and the predicted value based on the regression line) does not become a normal distribution, and there were numerous cases where the result of the regression calculation could not be used as the estimated value. Thus, the computer system performs Boxcox transformation to cause the residual error to be a normal distribution before the regression analysis is conducted, and subsequently obtains the estimated value of the corrected decay rate constant $\beta_g{}^{(A)}$.

The left-hand side of Math (5) obtained as $1-p_{g,i,w} = \lambda_{g,i,w}$ in Math (4) is Boxcox transformation of $[\mathrm{mRNA}_{g,i,w}]$. The likelihood upon assuming that the residual error in the regression after Boxcox transformation is following a normal distribution is given in Math (6). Accordingly, $p_{g,i,w}$ is obtained by deciding $\lambda_{g,i,w}$ so that the likelihood of Math (6) is maximized.

[Math 5]

$$\begin{cases} \dfrac{[\mathrm{mRNA}_{g,i,w}]^{\lambda_{g,i,w}}-1}{\lambda_{g,i,w}} = b_{g,i,w}{}^{(A)}t + a_{g,i,w}{}^{(A)}\,(\lambda_{g,i,w} \neq 0) \\ \log[\mathrm{mRNA}_{g,i,w}] = b_{g,i,w}{}^{(A)}t + a_{g,i,w}{}^{(A)}\,(\lambda_{g,i,w} = 0) \end{cases} \qquad (5)$$

[Math 6]

$$L = -\frac{n}{2}\log\left(y^{(\lambda)\mathrm{T}}\left(I - X(X^{\mathrm{T}}X)^{-1}X^{\mathrm{T}}\right)y^{(\lambda)}\right) + (\lambda - 1)\sum_{i=1}^{n}\log y_{t_i} \qquad (6)$$

[Math 7]

$$X = \begin{bmatrix} 1 & t_1 \\ \vdots & \vdots \\ 1 & t_n \end{bmatrix} \qquad (7)$$

X is shown in Math (7). I is the identity matrix. In Math (7), the mRNA amount at time ti is expressed as yti, the vector obtained by arranging values as a result of subjecting $y_{t_i}$ to Boxcox transformation is expressed as $y^{(\lambda)}$, and the dimension of $y^{(\lambda)}$ is expressed as n. Because there are hardly any reactions of a reaction order of 3 or higher, the search range of $\lambda_{g,i,w}$ was set from -2 to 2. The interval was set to 0.2. This search range is the same search range as scipy of Python.

The half-life in the model of Math (3) is given by $(-1/(\beta_g{}^{(A)}\lambda))*(1-2^{-\lambda})$. Thus, the system allows the user to select whether to output the decay rate constant or output the half-life.

The statistics function f for integrating a plurality of region-based decay rates $(b_{g,i,w}{}^{(A)})$ into a corrected decay rate constant $(\beta_g{}^{(A)})$ outputs the corrected decay rate constant $(\beta_g{}^{(A)})$ as a representative value based on the average value, median value, minimum absolute value, or maximum absolute value of a plurality of region-based decay rates, The system of the present invention enables the user to select from the calculation methods of five representative values; namely, the average value, median value, minimum absolute value, or maximum absolute value of a plurality of region-based decay rates in addition to the conventional method.

The definition of the statistics function f when the average value is selected is as per Math (8) and Math (9) below.

[Math 8]

$$f\left(b_{g,i,w}^{(A)}\right) = \sum_{\substack{i,w \\ b_{g,i,w}^{(A)} \neq 0}} b_{g,i,w}^{(A)}/N \qquad (8)$$

[Math 9]

$$N = \sum_{\substack{i,w \\ b_{g,i,w}^{(A)} \neq 0}} 1 \qquad (9)$$

The definition of the statistics function f when the median value is selected is as per Math (10) below, $x_k$ is a numerical sequence rearranged in order from the smallest value, excluding zero among $b_{g,i,w}^{(A)}$, and N is the same value as that of Math (9). [Math 10]

$$f\left(b_{g,i,w}^{(A)}\right) = x_{\frac{N+1}{2}} \qquad \text{(N IS ODD)}$$

$$f\left(b_{g,i,w}^{(A)}\right) = \frac{1}{2}\left(x_{\frac{N}{2}} + x_{\frac{N}{2}+1}\right) \qquad \text{(N IS EVEN)} \qquad (10)$$

*(N is an odd number)*
*(N is an even number)*

The definition of the statistics function f when the minimum absolute value is selected is as per Math (11) below [Math 11]

$$f\left(b_{g,i,w}^{(A)}\right) = \min_{b_{g,i,w}^{(A)} \neq 0} |b_{g,i,w}^{(A)}| \qquad (11)$$

The definition of the statistics function f when the maximum absolute value is selected is as per Math (12) below. [Math 12]

$$f\left(b_{g,i,w}^{(A)}\right) = \max_{b_{g,i,w}^{(A)} \neq 0} |b_{g,i,w}^{(A)}| \qquad (12)$$

In the conventional method, the region-based decay rate $b_{g,i,w}^{(A)}$ obtained by designating the entire gene region as the region (i = 0, w = gene length) is used as is as the corrected decay rate constant $\beta_g^{(A)}$. The definition of the statistics function f when the conventional method is selected is as per Math (13) below. $L_g$ is the length of the gene g. [Math 13]

$$f\left(b_{g,i,w}^{(A)}\right) = b_{g,1,L_g}^{(A)} \qquad (13)$$

The system according to Fig. 1 is explained once again. The controller of the calculation server 120 of Fig. 1 realizes the stability simulation of biopolymers based on the foregoing knowledge according to the program recorded in the memory. The client computer 100 described above comprises an RNA-Seq data reception module 102, a machine learning parameter reception module 104, and a machine learning result browsing module 106. Each of these modules is realized by the controller executing the programs recorded in the memory. The term "module" may otherwise be referred to as a means, function, unit, part or the like.

The RNA-Seq data reception module 102 receives from the user an RNA-Seq data request of mRNA of a prescribed gene g. The machine learning parameter reception module 104 sets, based on the user's input, the parameter for controlling the development of machine learning in the machine learning processing module 134 (described later) of the calculation server 120. The machine learning parameter reception module 104 receives inputs from the user using, for example, a keyboard, mouse, touch panel, numeric keypad, scanner, microphone, or sensor. Specifically, the machine learning parameter reception module 104 may also receive inputs via the screen shown in Fig. 5.

This parameter may be, as the index of stability of biopolymers, selection of decay rate constant or half-life, use or non-use of Boxcox transformation upon regressing the foregoing [$mRNA_{g,i,w}$] (mRNA amount of the region defined by i, w of the gene g at a certain time) over time, lower limit of the Ratio (described later), upper limit of the standard deviation (described later), or selection of the type (average value, intermediate value or the like) of the foregoing statistics function. The reason why the client computer 100 allows the user to set the parameter is to enable machine learning according to the characteristics of the gene or biopolymer (mRNA). The machine learning result browsing module 106 refers to the machine learning result database 124 and enables the user to browse the results of machine learning. The machine learning result browsing module 106 outputs the results, for example, via a display, printer, or speaker (Fig. 6).

The calculation server 120 executes the stability simulation system of biopolymers by coordinating with databases. As the databases, there are an RNA-Seq database 122 and a machine learning result database 124. The machine learning result database 124 stores summary data 12401 and a learning model 12402 related to machine learning. A database is, for example, a ROM (Read Only Memory), a RAM (Random Access Memory), an HDD (Hard Disk Drive), or a flash memory.

The calculation server 120 comprises a Depth calculation processing module 126, a region-based mRNA amount calculation processing module 128, a region-based decay rate calculation processing module 130, a corrected decay rate constant calculation processing module 132, and a machine learning processing module 134. These modules are also realized by the controller of the calculation server 120 executing programs. The combination of these modules configures the stability simulation module of biopolymers.

The Depth calculation processing module 126 executes data processing for creating the graph of Fig. 2 based on the time series data of the mRNA amount (Depth) at an arbitrary point of the mRNA sequence by referring to the RNA-Seq database 122 based on the gene mRNA received by the RNA-Seq data reception module 102.

The region-based mRNA amount calculation processing module 128 calculates the amount of mRNA ([$mRNA_{g,i,w}$] described above: mRNA amount of the region defined by i, w of the gene g at time t) for each region (Fig. 2: 200) of the mRNA base sequence based on the calculation result of Depth. The corrected decay rate constant calculation processing module 132 uses the statistics function f described above and integrates a plurality of region-based decay rates ($b_{g,i,w}^{(A)}$) into a corrected decay rate constant ($\beta_g^{(A)}$).

The machine learning processing module 134 constructs a prediction model based on the Least Absolute Shrinkage and Selection Operator (LASSO) by using, as the explanation variable, a squared term configured from the codon frequency of mRNA molecules and the logarithmic value of the CDS length and the 3'UTR length and their arbitrary combination. As the prediction model, Logistic regression, Ridge regression, Elastic Net regression or the like may also be used. The machine learning processing module 134 randomly assigns training data and test data, performs the operation of model creation and the operation of obtaining the correlation coefficient multiple times to obtain the average thereof, and outputs the average as the ultimate correlation coefficient. Specifically, the operation of obtaining the correlation coefficient may be performed 1000 times, and the coefficient obtained by calculating the average may be used as the correlation coefficient.

The machine learning processing module 134 calculates the amount related to the explanation variable using a custom Python script based on a GTF file. Python scikit-learn (v1.2.1) is used for the prediction model construction based on LASSO.

The present inventors tested the following two methods as the method of selecting the training gene set for use in constructing the learning model and the test gene set for use in the performance evaluation. In the first method, in the same manner as the conventional method, 65,977 training genes and 7,376 test genes were used. $\beta_g^{(S)}$ was set as the objective variable to construct the prediction model, and the correlation coefficient was 0.43.

The second method is for the performance validation based on Bootstrap. The process of randomly selecting 10% from 97,804 genes, which are the entire gene, as the test genes and constructing a prediction model was repeated 1000 times. The average of the obtained correlation coefficient was 0.12.

The vector in which learning gene names are arranged is set as $g_{train}$, and the vector obtained by deriving and arranging the decay rate constant $\beta_g^{(S)}$ according to Math (1), Math (2) and function f for each element g of gtrain is set as $\beta_{train}^{(S)(true)}$. These are used as the objective variable of the learning data of machine learning. Since the calculation method of the correlation coefficient will not change for $\beta_{train}^{(S)(true)}$ or $\beta_{train}^{(A)(true)}$, this is hereinafter indicated as $\beta_{train}^{(x)(train)}$.

Next, the machine learning processing module 134 calculates the feature quantity matrix $Q^{(train)}$ of the codon frequency or the like = p(Gtrain). $Q_{i,j}$ is the matrix having the value of the j-th feature quantity concerning the i-th gene, and shall be the explanation variable of the learning data of machine learning. p is the function of returning a matrix Q with the set of the gene name as the argument.

Next, the machine learning processing module 134 decides $f_{LASS0}^{(x)}$ to become $\beta_{train}^{(X)(true)} = f_{LASS0}^{(x)} (Q^{(train)})$ based on LASSO. Finally, the vector in which the test gene names are arranged is set as $g_{test}$, and the decay rate constant $\beta_{test}^{(x)(true)}$ is derived according to Math (1) and Math (2) or Math (3) to Math (7) and the statistics function f. Meanwhile, feature quantity $Q^{(test)} = p(G_{test})$ is calculated, and $\beta_{test}^{(X)(pred)} = f_{LASS0}^{(x)} (Q^{(test)})$ is obtained. Here, the correlation coefficient (r) is expressed with Math (14) below. Cov(•,•) is covariance, and V(•) is variance.
[Math 14]

$$r = \frac{\text{Cov}\left(\beta_{test}^{(x)(true)}, \beta_{test}^{(x)(pred)}\right)}{\sqrt{V\left(\beta_{test}^{(x)(true)}\right)V\left(\beta_{test}^{(x)(pred)}\right)}} \qquad (14)$$

The machine learning processing module 134 uses a reliable biopolymer (mRNA), and does not use unreliable genes as training data and test data, in order to improve the accuracy of machine learning. Thus, the system uses, for machine learning, mRNA in which the Ratio of the region where the value of the vertical axis of Fig. 2 is not zero (non-zero region) relative to the entire region is a prescribed threshold value or higher, and the standard deviation of the decay rate ($b_{wi}^{(A)}$) of the region-based rate regarding the entire region is a prescribed threshold or less.

The standard deviations (SD) calculated respectively based on the conventional method and the present invention - change characteristics of the correlation coefficient, are now explained upon comparison for each difference in the foregoing Ratio. Nevertheless, the ultimate correlation coefficient was obtained by randomly assigning training data and test data, performing the operation of model creation and the operation of obtaining the correlation coefficient 1000 times, and obtaining the average thereof.

As a result of machine learning, the correlation coefficient of the present invention improved in comparison to the conventional method. Fig. 3(A) shows the correlation coefficient when the statistics function f is "average value" - change characteristics for each Ratio of the standard deviation (SD) described above. Fig. 3(B) shows the correlation coefficient when the statistics function f is "median value" - change characteristics of the standard deviation (SD) described above. Fig. 3(C) shows the change characteristics in the conventional method, and SD is the standard deviation of $\beta_g^{(S)}$ described above. Upon comparing Fig. 3(A), Fig. 3(B) and Fig. 3(C), it can be understood that the correlation coefficient of the present invention is higher than the correlation coefficient of the conventional method.

The results upon comparing the correlation coefficient of the present invention and the correlation coefficient of the conventional method are shown in Fig. 4. Fig. 4 differs from Fig. 3 with respect to the point that Boxcox transformation is performed at the time of conducting regression analysis. Fig. 4(A) shows the correlation coefficient when the statistics function f is "average value" - change characteristics for each Ratio of the standard deviation (SD) described above. Fig. 4(B) shows the correlation coefficient when the statistics function f is "median value" - change characteristics of the standard deviation (SD) described above. Fig. 4(C) shows the change characteristics in the conventional method. Upon comparing Fig. 4(A), Fig. 4(B) and Fig. 4(C), it can be understood that the correlation coefficient of the present invention is a further improvement of the correlation coefficient of the conventional method.

The operation of mRNA stability simulation using the learning model 12402 is now explained. The user inputs a prescribed nucleotide into the client computer 100. This information is sent to the calculation server 120, and the simulation analyzing unit of the calculation server 120 refers to the machine learning result database 124 and decides a plurality of nucleotide sequence candidates having the same amino acid sequence coded by the nucleotide sequence.

The simulation analyzing unit inputs, into the learning model 12402, sequence information of each of a plurality of candidates as an explanation variable. The learning model 12402 calculates an objective variable based on the explanation variable of each of a plurality of candidates, and outputs, in a ranking format, a plurality of candidates in order from those having a high decay rate constant. The user can decide the mRNA having a sequence with the most superior degradation stability based on this ranking.

According to the embodiment described above, provided is a first computer system which simulates stability of a biopolymer, comprising: a controller that executes a program stored in a memory, wherein the controller: acquires information of a sequence of the biopolymer; classifies the sequence into a plurality of regions; calculates stability of each of the plurality of regions; and executes predictive simulation of the stability of the biopolymer based on a representative value as a representation of the stability of each of the plurality of regions based on a result of the calculation. According to the first computer system, it is possible to predict, with high accuracy, the degradation stability of biopolymers having an arbitrary sequence.

Furthermore, according to the embodiment described above, provided is a second computer system, which in the first computer system, extracts each of the plurality of regions from the sequence of the biopolymer by identifying a location and a width of the sequence; and calculates the stability of each of the plurality of regions based on time series data of an amount of the biopolymer. According to the second computer system, it is possible to obtain the stability of each of the plurality of regions.

Furthermore, according to the embodiment described above, provided is a third computer system, which, in the second computer system, calculates the stability of each of the plurality of regions based on a rate at which the sequence of the corresponding region becomes decayed. According to the third computer system, it is possible to accurately obtain the stability of each of the plurality of regions.

Moreover, according to the embodiment described above, provided is a fourth computer system which, in the first computer system, executes machine learning which uses the stability of the biopolymer itself as an objective variable and uses sequence information configuring the biopolymer as an explanation variable; and constructs a prediction model of the stability of the biopolymer itself based on the machine learning. According to the fourth computer system, it is possible to provide a learning model capable of accurately predicting the stability of biopolymers.

Moreover, according to the embodiment described above, provided is a fifth computer system which, in the fourth computer system, newly acquires information of a sequence of a biopolymer; and calculates the stability of the biopolymer itself based on the prediction model. According to the fifth computer system, even when a biopolymer is newly received as an input, it is possible to accurately calculate the stability of the newly received biopolymer based on the constructed prediction model.

Moreover, according to the embodiment described above, provided is a sixth computer system which, in the third computer system, calculates a rate at which the sequence of the region becomes decayed from the amount of the biopolymer having the sequence of the corresponding region based on the time series data of the biopolymer. According to the sixth computer system, it is possible to accurately calculate the stability of the region.

Moreover, according to the embodiment described above, provided is a seventh computer system which, in the fifth computer system, receives, as an input, a method of calculating the representative value. According to the seventh computer system, the user can suitably change the method of obtaining the representative value according to the biopolymer.

According to the embodiment described above, provided is an eighth computer system which, in the first computer system, receives time series data of mRNA as the biopolymer; calculates region-based corrected stability of a sequence of the mRNA from the time series data; calculates corrected stability as an objective variable in machine learning based on the stability; generates a learning model by performing machine learning based on the corrected stability and the time series data; and outputs the learning model. According to the eighth computer system, it is possible to improve the prediction accuracy of mRNA stability.

Furthermore, according to the embodiment described above, provided is a ninth computer system which, in the second computer system, calculates, for each of the plurality of regions, stability of each of the regions by regressing a biopolymer amount having the sequence of the corresponding region over time. According to the ninth computer system, it is possible to estimate the stability of each of the regions based on regression analysis.

Furthermore, according to the embodiment described above, provided is a tenth computer system which, in the fifth computer system, executes, when regressing a biopolymer amount having the sequence of the corresponding region over time, Boxcox transformation to the biopolymer amount based on a user's input. According to the tenth computer system, the user can select whether to perform Boxcox transformation according to the attribute of the biopolymer.

Furthermore, according to the embodiment described above, provided is an eleventh computer system which simulates stability of a biopolymer, comprising: a controller that executes a program stored in a memory, wherein the controller: receives time series data of mRNA as the biopolymer; classifies the sequence into a plurality of regions; calculates stability of each of the plurality of regions; and outputs the stability. According to the eleventh computer system, it is possible to predict, with high accuracy, the degradation stability of biopolymers having an arbitrary sequence.

Furthermore, according to the embodiment described above, provided is a method of simulating stability of a biopolymer, wherein the method comprises the steps of a computer: acquiring information of a sequence of the biopolymer; classifying the sequence into a plurality of regions; calculating stability of each of the plurality of regions; and executing predictive simulation of the stability of the biopolymer itself based on a representative value as a representation of the stability of each of the plurality of regions based on a result of the calculation.

While embodiments of the present invention have been explained above, the technical scope of the present invention is not limited to the scope described in the foregoing embodiments. It is obvious that, based on the wording of the claims, the various modifications and improvements in the foregoing embodiment are also included in the technical scope of the present invention.

For example, while the foregoing embodiments explained a case of taking mRNA as the biopolymer, the present invention can also be applied to types that have different stability for each region of the monomer sequence of biopolymers, such as proteins and polysaccharides which have different stability depending on the steric structure.

REFERENCE SIGNS LIST

100: client computer, 102: RNA-Seq data reception module, 104: machine learning parameter reception module, 106: machine learning result browsing module, 120: calculation server, 122: RNA-Seq database, 124: machine learning result database, 126: Depth calculation processing module, 128: region-based mRNA amount calculation processing module, 130: region-based decay rate calculation processing module, 132: corrected decay rate constant calculation processing module, 134: machine learning processing module, 200: region, 12401: machine learning summary data, 12402: learning model

## Claims

1. A computer system which simulates stability of a biopolymer, comprising:

   a controller that executes a program stored in a memory,
   wherein the controller:

   acquires information of a sequence of the biopolymer;
   classifies the sequence into a plurality of regions;
   calculates stability of each of the plurality of regions; and
   executes predictive simulation of the stability of the biopolymer based on a representative value as a representation of the stability of each of the plurality of regions based on a result of the calculation.

2. The computer system according to claim 1,
   wherein the controller:

   extracts each of the plurality of regions from the sequence of the biopolymer by identifying a location and a width of the sequence; and
   calculates the stability of each of the plurality of regions based on time series data of an amount of the biopolymer.

3. The computer system according to claim 2,
   wherein the controller:
   calculates the stability of each of the plurality of regions based on a rate at which the sequence of the corresponding region becomes decayed.

4. The computer system according to claim 1,
   wherein the controller:

   executes machine learning which uses the stability of the biopolymer as an objective variable and uses sequence information configuring the biopolymer as an explanation variable; and
   constructs a prediction model of the stability of the biopolymer itself based. on the machine learning.

5. The computer system according to claim 4,
   wherein the controller:

   newly acquires information of a sequence of a biopolymer; and
   calculates the stability of the biopolymer itself based on the prediction model.

6. The computer system according to claim 3,
   wherein the controller:
   calculates a rate at which the sequence of the region becomes decayed from the amount of the biopolymer having the sequence of the corresponding region based on the time series data of the biopolymer.

7. The computer system according to claim 6,
   wherein the controller:
   receives, as an input, a method of calculating the representative value.

**8.** The computer system according to claim 1,
wherein the controller:

receives time series data of mRNA as the biopolymer;
calculates region-based stability of a sequence of the mRNA from the time series data;
calculates corrected stability as an objective variable in machine learning based on the stability;
generates a learning model by performing machine learning based on the corrected stability and the time series data; and
outputs the learning model.

**9.** The computer system according to claim 2,
wherein the controller:
calculates, for each of the plurality of regions, stability of each of the regions by regressing a biopolymer amount having the sequence of the corresponding region over time.

**10.** The computer system according to claim 6,
wherein the controller:
executes, when regressing a biopolymer amount having the sequence of the corresponding region over time, Boxcox transformation to the biopolymer amount based on a user's input.

**11.** A computer system which simulates stability of a biopolymer, comprising:

a controller that executes a program stored in a memory,
wherein the controller:

receives time series data of mRNA as the biopolymer;
classifies the sequence into a plurality of regions;
calculates stability of each of the plurality of regions; and
outputs the stability.

**12.** A method of simulating stability of a biopolymer,
wherein the method comprises the steps of:

acquiring information of a sequence of the biopolymer;
classifying the sequence of the biopolymer into a plurality of regions;
calculating stability of each of the plurality of regions; and
executing predictive simulation of the stability of the biopolymer itself based on a representative value as a representation of the stability of each of the plurality of regions based on a result of the calculation.

## FIG. 1

FIG. 2

# FIG. 3

## FIG. 4

# FIG. 5

# FIG. 6

600

| MACHINE LEARNING RESULT | | | | |
|---|---|---|---|---|
| LEARNING MODEL ID | RNA FILE NAME | GENE NUMBER | CORRELATION COEFFICIENT | DOWNLOAD BUTTON |
| 646T-0001 | XXXX | 9453 | 0.6152 | Push |
| 646T-0002 | XXXX | 8577 | 0.5564 | Push |
| . | . | . | . | . |
| | . | . | . | |
| . | . | . | . | . |

Europäisches
Patentamt

European
Patent Office

Office européen
des brevets

# EUROPEAN SEARCH REPORT

Application Number

EP 24 16 0954

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WAYMENT-STEELE HANNAH K. ET AL: "Deep learning models for predicting RNA degradation via dual crowdsourcing", NATURE MACHINE INTELLIGENCE, [Online] vol. 4, no. 12, 14 December 2022 (2022-12-14), pages 1174-1184, XP093186439, ISSN: 2522-5839, DOI: 10.1038/s42256-022-00571-8 Retrieved from the Internet: URL:https://www.nature.com/articles/s42256-022-00571-8> [retrieved on 2024-07-16] * the whole document * * in particular * * "Methods" and "Results" section, reference [13] (Leppek et. al); figures 1-3, 5 * | 1-12 | INV. G16B15/00 G16B40/20 G16C20/30 G16C20/70 |

----- 

| | | | TECHNICAL FIELDS SEARCHED (IPC) |
|---|---|---|---|
| | | | G16B G16C |

The present search report has been drawn up for all claims

| Place of search | Date of completion of the search | Examiner |
|---|---|---|
| Munich | 16 July 2024 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023078036 A

**Non-patent literature cited in the description**

- **MEDINA-MUNOZ ; SANTIAGO GERARDO et al.** Crosstalk between codon optimality and cis-regulatory elements dictates mRNA stability. *Genome biology,* 2021, vol. 22 (1), 1-23